(19) Europäisches Patentamt
European Patent Office
Office européen des brevets

(11) Publication number: **0 164 959 B1**

(12) # EUROPEAN PATENT SPECIFICATION

(45) Date of publication of patent specification: **11.03.92**

(51) Int. Cl.5: **A61K 9/54**, A61K 31/44

(21) Application number: **85303793.5**

(22) Date of filing: **30.05.85**

The file contains technical information submitted after the application was filed and not included in this specification

(54) **Pharmaceutical composition in sustained release unit dose form and process for its preparation.**

(30) Priority: **04.06.84 GB 8414220**

(43) Date of publication of application:
**18.12.85 Bulletin 85/51**

(45) Publication of the grant of the patent:
**11.03.92 Bulletin 92/11**

(84) Designated Contracting States:
**AT BE CH DE FR IT LI LU NL SE**

(56) References cited:
**CA-A- 1 200 494**
**FR-A- 2 313 915**
**GB-A- 2 065 642**
**JP-A-58 116 414**
**LU-A- 85 290**

**CHEMICAL ABSTRACTS, vol. 105, no. 4, 28th July 1986, page 386, abstract no. 30065d, Columbus, Ohio, US; & CA - A - 1 200 494 & JP - A - 58 116 414 (T. SONOBE et al.) 11-02-86**

(73) Proprietor: **STERWIN AG.**
**Zeughausgasse 9**
**CH-6300 Zug(CH)**

(72) Inventor: **Harrison, Paul Jonathan**
**7, Stott Street**
**Alnwick Northumberland(GB)**
Inventor: **Potter, Christopher John**
**2, Garden Terrace Whittingham**
**Alnwick Northumberland NE66 4RD(GB)**
Inventor: **Langridge, John Richard**
**29, Arkle Court**
**Alnwick Northumberland(GB)**

(74) Representative: **Bankes, Stephen Charles Digby et al**
**BARON & WARREN 18 South End Kensington**
**London W8 5BU(GB)**

EP 0 164 959 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a sustained release form of a medicament for administration by the oral route.

The use of enteric coatings on medicaments in order that the medicaments shall pass through a patient's stomach unchanged and thus ensure that the active ingredient or ingredients are released in the patient's small intestine where the pH is normally between 5.5 and 7.5 is now an established method of treatment. This prevents irritation of the gastrointestinal tract and is often convenient as it may make it unnecessary for a patient to take a dose of medicament more often than two or three times a day to maintain effective blood levels of medicament. A substantial number of synthetic polymeric materials have been proposed for use in such formulations and the nature of the coatings used in the formulations have varied considerably depending upon the results sought. Thus the synthetic polymeric materials used have included polymers of vinyl monomers such as vinyl pyrrolidone and vinyl acetate phthalate and the semi-synthetic derivatives of celluloses such as cellulose ethers and carboxycelluloses, e.g. cellulose acetate phthalate and hydroxypropylmethyl cellulose phthalate.

In come cases partial solution of a medicament in the patient's stomach is required especially if gradual solution in both the stomach and the small intestine is the desirable course to aim at. This presents problems because of the differences in the pH values prevailing in the stomach and the intestine, and in the differences in the chemical and physical properties of particular medicaments when submitted to these differing pH conditions. In general the differences are most difficult to overcome with medicaments containing one or more amino groups in the molecule. Individual solutions have to be found to the particular problems posed by each system.

In two or three cases in which the medicament is only soluble at pH values between 1 and 4 solution can take place naturally in the stomach but does not occur at all in the small intestine. To overcome this difficulty it has been proposed to include a readily soluble pharmacologically acceptable acid in the inner or core portion of each unit of medicament: the amount of this acid may be two or more molecules for each molecule of medicament and it enables sufficiently acid conditions to be set up locally in the small intestine for the medicament to dissolve and be absorbed through the walls of the intestine. The core is surrounded by a semipermeable coating containing a mixture of film-forming materials one of which is soluble and the other of which is insoluble in the gastric juices (see US-A-4 361 546, 4 367 2 17 and 4 438 091). It will be appreciated that in this way a gradual release of medicament can be brought about and consequentially there is gradual absorption through the walls of the stomach and the small intestine. However it is limited to the particular solubility characteristics indicated for the medicament.

JP-A-58116414 discloses preparations of nicardipine, which has high solubility in gastric juices and very low solubility in the intestine. The composition is in the form of spherical pellets comprising small granular nuclei spray-coated with a solution comprising (a) nicardipine or a salt thereof and (b) a pH-dependent additive and/or a watersoluble polymer in an organic solvent, a mixture of organic solvents or a mixture of water and an organic solvent.

A different problem arises when a medicament has a high solubility in the low pH gastric juices and a very much lower solubility in the higher pH intestinal juices, which lower solubility may nevertheless be sufficient for a sustained release formulation. We have now encountered a group of medicaments in which such solubility characteristics have been found to exist and for which a sustained release formulation is required.

IN GB-A-2 065 642 there are described a number of 5-(pyridinyl)-2(1H) pyridones which are reported to be useful as cardiotonic agents. Certain of these compounds have been found to show promise for use in vivo and to be potential materials for use with human patients but they have one important drawback viz that they are very readily eliminated from the human system as demonstrated by the plasma profiles obtained after administration to human patients. These compounds have been found to have much greater solubility in the gastric juices at pH 1.5 than they have at pH 4.5. In one instance the solubility is substantially fifty times greater at pH 1.5 than it is at pH 5 to 8.

It is accordingly an object of this invention to provide a sustained release form of the above mentioned medicaments and others that are orally administered and have a high solubility in gastric juices but are very readily eliminated from the human system, which form will overcome this drawback.

Accordingly the invention consists in a pharmaceutical composition of a medicament with the solubility pattern described in sustained release unit dosage form for oral administration, comprising a plurality of beads within a closed capsule of a gastric juice-soluble material, characterised in that each said bead has an inert particulate core having adhered thereto a coating of particles of said medicament, said coating of medicament being surrounded by a sustaining coating comprising at least three admixed polymers, a first said polymer being soluble in gastric juices at all pH values encountered in the gastrointestinal tract, a

second said polymer being substantially insoluble in gastric juices at pH values below 3 but soluble therein at pH values of 5 and above and the third said polymer being insoluble in the contents of the gastrointestinal tract at all pH values normally encountered therein, and the three polymers being present in such proportions as to permit a sustained release of the medicament during passage of the beads through the stomach and the intestinal tract.

It is preferred that the weight of the polymer which is insoluble in the contents of the gastrointestinal tract is greater than the sum of the weights of the other two polymers present in the sustaining coating. A convenient ratio of the weight of the insoluble polymer to the combined weights of the other two polymers present in the sustaining layer has been found to be from 3 : 2 to 2 : 1.

The invention has been found to have a particular application to the formulation in unit dosage form for administration by the oral route of pyridyl-(1H) pyridones having the general formula

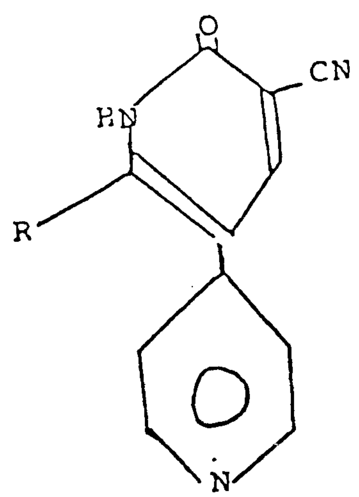

(I)

in which R is an alkyl group having 1 to 4 carbon atoms. With such materials it has been found that upon administration by the oral route the concentration of the medicament in the plasma rises very rapidly during the first hour and then falls by approximately two-thirds of the maximum reached during the second hour. Subsequently it falls at a somewhat diminishing rate during the third and subsequent hours. If a single dose is to be sufficient to maintain effective blood levels in a patient for a substantial number of hours e.g. 4 or 8 hours a system needs to be devised in which only a portion of the dosage is made available for absorption into the blood at any one time. Continual release of the medicament will maintain effective blood levels until the next dose of medication is taken. The rate of dissolution (and therefore availability) has been found to be determined by the pH of the particular part of the gastrointestinal tract.

It has been found that in the case of the pyridyl-pyridones the rate of dissolution is greatest in the range of lowest pH value which is in the stomach and the rate of dissolution decreases as the pH rises along the passage of the upper gastrointestinal tract.

Consequently the polymers used and the proportions of these in the sustaining layer will determine the release characteristics of the medicament from the dosage form.

We prefer to use nonpareils as the inert substrate material of the beads that we prepare.

The substrate is then coated with particles of the medicament in solid form. It may be necessary to convert a medicament to a derivative such as a salt in order to obtain it in solid form. Medicaments available in solid form may need to be ground in order to obtain particles sufficiently small to be conveniently adhered to the particles of core material. The latter are conveniently of a size which will pass a 25 US standard mesh (707 µm) and be retained on a 30 US standard mesh (595 µm). To adhere the particles of solid medicament to the inert substrate we prefer to use a water soluble pharmacologically acceptable adhesive such as a suitable grade of hydroxypropylmethylcellulose. The hydroxypropylmethylcellulose used may be that known as "Pharmacoat 606", a 6-centipoise grade of hydroxypropylmethylcellulose. A thorough dispersion of the solid medicament in Pharmacoat 606 solution is then prepared and used to coat the nonpareils or other particulate inert substrate material in a coating column and dry the coated material at a raised temperature, e.g. 60 ° C.

The sustaining coating essentially contains three polymers each of which behaves differently in the gastrointestinal tract. All three polymers may be cellulose derivatives and each of the polymers may be a mixture. However, whether each be a single individual or a mixture it must conform to certain solubility

requirements in relation to the gastrointestinal tract.

The first polymer should be soluble in the gastric juices at all pH values encountered in the stomach and the intestine. In the case of the pyridyl pyridones this includes the pH range over which these substances exhibit their maximum solubility in the gastric juices:
when this is the case the preferred polymer is hydroxypropylmethylcellulose. Other polymers which may be used for this purpose include polyvinylpyrrolidone and sodium carboxymethylcellulose. When it is essential to reduce the rate of dissolution of the medicament at pH values of the order of 1.5 the proportion of this polymer in the mixture of polymers should be kept low e.g. 15% - 20% or less by weight of the whole mixture of polymers.

The second polymer used is one which is substantially insoluble in gastric juices at pH values below 3 but soluble therein at pH values of 5 and above. The use of such a polymer ensures that while this part of the coating remains substantially intact at the pH values normally encountered in the stomach, typically 1.5 - 2.0, at pH 5 and above the permeability of the coating to the medicament increases and this rise in permeability counteracts the reduced solubility of the medicament to reduce the pH dependence of the release rate. This polymer may start to become soluble at pH values lower than 5, for example at 3.5 or 4. The preferred polymer for this purpose is hydroxypropylmethylcellulose phthalate. Other polymers which are suitable for this purpose include copolymers of the lower alkyl methacrylates and polyvinylacetate phthalate.

The third polymer used should be one which is insoluble at all pH values normally encountered in the gastrointestinal tract. In the lower gastrointestinal tract pH values of about 7.5 are normally to be expected and this is the minimum value for insolubility of the third polymer. The preferred third polymer is ethyl cellulose. Other polymers which may be used include copolymers of the lower alkyl methacrylates in which the copolymerising monomer contains a hydrophilic group.

Other factors which affect the rate of release of the medicament present include the thickness of the sustaining coating and the ratios of the three polymers present in the sustaining coating. Regarding thickness of the coating the thicker the coating the slower the rate of release at all pH values.

The polymer ratios have an important bearing upon the rate of release of medicament at all pH values. Increase in the ratio of the first polymer to the third polymer raises the rate of release of medicament at low pH values, i.e, in the stomach whilst decrease in this ratio reduces the rate of release. Increase in the ratio of the second polymer to the third polymer increases the rate of release at pH values above about 5. Increase in the ratio of the second polymer to the first polymer without changing the proportion of the third polymer increases the rate of release at pH values above about 5 and decreases the rate of release at pH values below about 5.

According to a further aspect of the invention there is provided a process for producing a pharmaceutical composition as defined above wherein the beads are prepared by coating inert core particles with a suspension of particles of the medicament and a binder for adhering said medicament particles to said core particles, and applying to said coated core particles a sustaining coating solution comprising at least the three polymers of differential solubility defined above.

In producing the unit dosage form of the product in accordance with the invention one may, for example, add 18 parts by weight of the three selected polymers to 261 parts by weight of a dispersion medium therefor. When the three polymers are cellulose ethers and ether esters, ethanol is a suitable medium. The resulting mixture is stirred until well dispersed and a low boiling solvent (e.g. methylene chloride) is then added and stirring continued until a clear solution is obtained. Nonpareils coated with medicaments are placed in a coating column or pan and the solution of the three polymers is then gradually fed into the column or pan whilst passing a current of warm air through the nonpareils until dry coated nonpareils are obtained.

The dried coated nonpareils are then weighed into unit dosage quantities and separate weighed quantities are fed into hard gelatine capsules and each capsule closed.

The following examples illustrate the invention. All parts are by weight.

PREPARATION 'A'

Production of Nonpareils coated with medicament

11 parts hydroxypropylmethylcellulose (Pharmacoat 606) are suspended in 111 parts of purified water previously heated to boiling. 440 additional parts of water are then added to the suspension and the whole stirred until a diluted Pharmacoat suspension has formed.

11 parts of 1,2-dihydro-6-methyl-2-oxo-5-(4-pyridinyl) -nicotinylnitrile are stirred into the Pharmacoat

supension until well dispersed. 200 parts of nonpareils (sucrose base passing a 25 US standard standard mesh (707 µm) and being retained on a 30 US standard mesh (595 µm)) are placed in a coating column or pan and whilst passing an atomizing current of warm air therethrough gradually feed in the diluted Pharmacoat suspension. After all the Pharmacoat suspension has been added continue the passage of the current of warm air until the coated nonpareils are dry.

EXAMPLE 1

There are placed in a suitable container 261 parts of ethanol, 11.70 parts of ethyl cellulose, 3.60 parts of hydroxypropylmethylcellulose and 2.70 parts of hydroxypropylmethylcellulose phthalate. The solids are stirred in until well dispersed and there is then added to the dispersion 621 parts of methylene choride. A clear solution should result.

Into a coating column or pan there are placed 222 parts of coated nonpareils prepared as described under Preparation A. Whilst passing an atomising current of warm air through the column the clear solution above described is gradually fed into the coating column or pan. After all the solution has been introduced into the column or pan passage of warm air is continued until the nonpareils are dry.

The product consisting of nonpareils first coated with medicament and then coated with sustaining coating of three polymers is then removed from the column and after cooling to room temperature, weighed out into portions each containing the required quantity of medicament which are separately fed into standard hard gelatin capsules and closed.

EXAMPLE 2

272.72 parts of nonpareils (passing a 25 US standard mesh (707 µm) and being retained on a 30 US standard mesh (595 µm)) were coated with a dispersion prepared from 15.0 parts of the same nitrile and 15.0 parts of hydroxypropylmethylcellulose (6 mPas (6 centipoises)) as described in Preparation A.

A sustaining coating solution is prepared from 6 parts of ethylcellulose, 2 parts of hydroxypropylmethylcellulose 6 mPas (6 centipoises)) and 2 parts of hydroxypropylmethylcellulose phthalate and used to coat the already coated nonpareils as described in Example 1. The subsequent procedure is also as described in Example 1.

EXAMPLE 3

Nonpareils are coated with nitrile as described in Example 2. A sustaining coating solution is then prepared from 12.42 parts of ethylcellulose, 4.14 parts of hydroxypropylmethylcellulose (6 mPas (6 centipoises)) and 4.14 parts of hydroxypropylmethylcellulose phthalate and the subsequent procedure is then as described in Example 1.

EXAMPLE 4

Nonpareils are coated with nitrile as described in Example 2. A sustaining coating solution is then prepared from 15.95 parts of ethylcellulose, 4.91 parts of hydroxypropylmethylcellulose and 3.68 parts of hydroxypropylmethylcellulose phthalate and the subsequent procedure is then as described in Example 1.

EXAMPLE 5

114 parts of nonpareils (passing a 25 US standard mesh (707 µm) ) and being retained on a 30 US standard mesh (595 µm)) were coated with a dispersion prepared from 15 parts of the same nitrile and 6.0 parts of hydroxypropylmethylcellulose (6 mPas (6 centipoises)) as described in Preparation A. A sustaining coating solution is prepared from 5.63 parts of ethylcellulose, 1.88 parts of hydroxypropylmethylcellulose (6 mPas or centipoises) and 1.88 parts of hydroxypropylmethylcellulose phthalate and used to coat the already coated nonpareils as described in Example. 1. The subsequent procedure is also as described in Example 1.

EXAMPLE 6

Nonpareils are coated with nitrile as described in Example 5. A sustaining coating solution is then prepared from 6.0 parts of ethylcellulose, 1.90 parts of hydroxypropylmethylcellulose (6 mPas (6 centipoises)) and 2.10 parts of hydroxypropylmethylcellulose phthalate. The subsequent procedure is then as described in Example 1.

Other nitriles having the general formula I have been prepared in sustained form by proceeding in the same manner as that illustrated in the above examples and the method is applicable to other solid medicaments having an elimination half-life of the order of 0.5 to 4 hours that can be applied to a core such as a nonpareil. In addition to cores formed of one or more normally crystalline sugars, with or without cellulose, inorganic materials such as calcium phosphate may be used as the core material.

The availability of sustained release formulations in accordance with this invention is of great assistance to the patient since it means that a patient does not need a unit dosage as frequently as would otherwise be the case to maintain effective blood levels of medicament. This minimises the risk of omission to take a dose at the correct time as well as avoiding the need to take a dose during the night.

The action of the controlled in vivo release resulting from the use of the formulations in accordance with the invention results in controlled and reproducible therapy by avoiding peak and trough periods in the plasma levels of patients taking the prescribed medicament. Such peaks and troughs are otherwise readily observable with a medicament having as short an elimination half-life period as 1 or 3 hours. A continuous release of medicament during passage through the stomach and the gastrointestinal tract is secured by the use of three polymers as described and this is effected in a simple coating operation.

The products of the present invention have been compared with conventional caplets containing an equal total weight of 1.12-dihydro-6-methyl-2-oxo-5-(4 pyridinyl-nicotinylitrile) (Compound A) in order to determine the bioavailability of the compound when administered to a patient in those forms. The products of the present invention were made up using nonpareils as the core material so that each capsule contained

| | |
|---|---|
| Compound A | 15. 0 mg |
| Pharmacoat 606 | 7. 9 mg |
| Nonpareils (25-30 US mesh) (595 - 707 µm) | 114. 0 mg |
| Ethyl cellulose | 6. 00 mg |
| HP-50 (hydroxypropylmethyl cellulose phthalate | 2. 10 mg |

The total weight of the filling for each capsule shell was 145.0 mg and this contained 15.0 mg of Compound A.

The conventionally formulated caplets respectively contained 5 mg and 10 mg of Compound A, one of each being administered to provide the reference quantity of Compound A. The compositions of the two caplets were as follows:

| | | |
|---|---|---|
| **Core:** | | |
| Compound A | 10 mg | 5 mg |
| Lactose Excipient | 209 mg | 104.5 mg |
| Pre-gelatinized starch | 80 mg | 40 mg |
| Microcrystalline cellulose AVICEL | 100 mg | 50 mg |
| Magnesium stearate | 1 mg | 0.5 mg |
| Core total: | 400 mg | 200 mg |
| **Coating:** | | |
| Hydroxypropyl-methylcellulose | 8.33 mg | 3.7 mg |
| Glyceryl triacetate | 1.67 mg | 0.739 mg |
| Titanium Dioxide | 0.265 mg | 1.480 mg |
| Quinolone Yellow Lake | 0.175 mg | 0.0704 mg |
| Erythrosine Lake | 0.060 mg | |
| Indigo Carmine Lake | - | 0.0131 mg |
| Total | 410. 5 mg | 206.0 mg |

The conventional caplets and capsules were given to a number of volunteers and the concentrations of Compound A in the plasma of the volunteers at various time intervals from 0.17 to 24 hours from the time of administration were determined. Graphs were prepared from the results obtained. An interval of one week was allowed between the first and second treatments for each volunteer.

Samples of plasma were taken from each volunteer at 10, 20, 30 and 45 minutes during the first hour after administration, then at half hourly intervals for 1 to 4 hours and then at 5, 6, 8, 11, 14 and 24 hours after administration. Parameters determined included maximum drug concentration in the plasma ($C_{max}$), time to reach maximum concentration ($t_{max}$). From the graphs drawn up the area under the curves of plasma concentration against time up to the last point of sampling was calculated using the trapezoidal rule (AUC). The graphs provided plasma profiles for the several test formulations from which the following mean data were read:

| | Cmax ng/ml | Tmax Time (h) |
|---|---|---|
| Conventional caplets | 422 | 0.67 |
| Capsules | 138 | 2.95 |

The plasma profiles with capsules were much flatter and broader than those obtained with caplets.

The mean relative bioavailability was 92%. This figure is based upon the areas AUC under the graph determined as outlined above.

The number of volunteers for whom the bioavailability was at least 75% of that obtained from caplets was 10 out of 10 in the case of capsules. The 75% figure is regarded as a criterion for a satisfactory sustained release formulation and it is apparent that this is consistently obtained in the case of the capsules. No adverse reactions were reported by volunteers to whom a capsule had been given. It thus

becomes apparent that capsules are a very satisfactory way of formulating materials having high solubility in gastric juices and lower, but nevertheless appreciable, solubility in the juices present in the small intestine to obtain a sustained release form.

## Claims

1. A pharmaceutical composition of a medicament in sustained release unit dosage form for oral administration, comprising a plurality of beads within a closed capsule of a gastric juice-soluble material, characterized in that the medicament has sufficient solubility in intestinal juices for sustained release therein and substantially higher solubility in the gastric juices, that each said bead has an inert particulate core having adhered thereto a coating of particles of said medicament, said coating of medicament being surrounded by a sustaining coating comprising at least three admixed polymers, a first said polymer being soluble in gastric juices at all pH values encountered in the gastrointestinal tract, a second said polymer being substantially insoluble in gastric juices at pH values below 3 but soluble therein at pH values of 5 and above and the third said polymer being insoluble in the contents of the gastrointestinal tract at all pH values normally encountered therein, and the three polymers being present in such proportions as to permit a sustained release of the medicament during passage of the beads through the stomach and the intestinal tract.

2. A pharmaceutical composition according to claim 1, characterized in that the weight of the insoluble third polymer in the sustaining coating is greater than the sum of the weights of the other two said polymers.

3. A pharmaceutical composition according to claim 2, characterized in that the ratio of the weight of the third polymer to the sum of the weights of the other two polymers is from 3:2 to 2:1.

4. A pharmaceutical composition according to any preceding claim, characterised in that said first polymer constitutes 20 wt.% or less of the polymer mixture forming the sustaining coating.

5. A pharmaceutical composition according to any preceding claim, characterized in that the medicament is a pyridyl-(1H)-pyridone having the general formula:

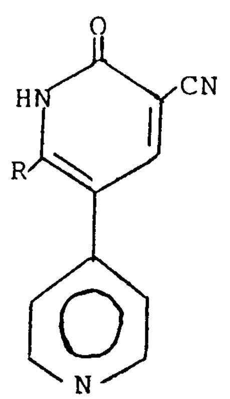

(I)

wherein R is an alkyl group having 1 to 4 carbon atoms, or a solid salt thereof.

6. A pharmaceutical composition according to any preceding claim, characterized in that the inert cores of the beads are in the form of nonpareils.

7. A pharmaceutical composition according to any preceding claim wherein the cores of the beads are of a mesh size not exceeding 707 µm.

8. A pharmaceutical composition according to claim 7, wherein the cores of the beads are of a mesh size from 595 to 707 µm.

9. A pharmaceutical composition according to any preceding claim, characterized in that the said first

polymer is selected from hydroxypropylmethylcellulose and polyvinylpyrrolidone.

**10.** A pharmaceutical composition according to any preceding claim, characterized in that said second polymer is hydroxypropylmethylcellulose phthalate.

**11.** A pharmaceutical composition according to any preceding claim, characterized in that said third polymer is ethyl cellulose.

**12.** A process for producing a pharmaceutical composition of a medicament in sustained release unit dosage form for oral administration, comprising a plurality of beads within a closed capsule of a gastric juice-soluble material, characterized in that the medicament has sufficient solubility in intestinal juices for sustained release therein and substantially higher solubility in gastric juices and that said beads are prepared by:

coating inert core particles with a suspension of particles of said medicament and a binder for adhering said medicament particles to said core particles and

applying to said coated core particles a sustaining coating solution comprising at least three admixed polymers, a first said polymer being soluble in gastric juices at all pH values encountered in the gastrointestinal tract, a second said polymer being substantially insoluble in gastric juices at pH values below 3 but soluble therein at pH values of 5 and above and the third said polymer being insoluble in the contents of the gastrointestinal tract at all pH values normally encountered therein, and the three polymers being present in such proportions as to permit a sustained release of the medicament during passage of the beads through the stomach and the intestinal tract.

**13.** A process according to claim 12, characterized in that said sustaining coating is formed by applying to the medicament-coated core particles a solution of said three polymers in a volatile solvent therefore and evaporating the solvent from the particles thus coated.

**14.** A process according to claim 13, characterized in that said solution is produced by forming a dispersion of the three polymers in a suitable medium, adding a low boiling solvent to the dispersion and stirring to give a clear solution.

**15.** A process according to claim 13 or claim 14, characterized in that the sustaining coating is applied by placing the medicament-coated core particles in a coating column or pan and feeding the polymer solution into the column or pan while passing a current of air through the particles to produce dry coated beads.

**16.** A process according to any one of claims 12 to 15, characterized in that the medicament is a pyridyl-(1H)-pyridone having the general formula:

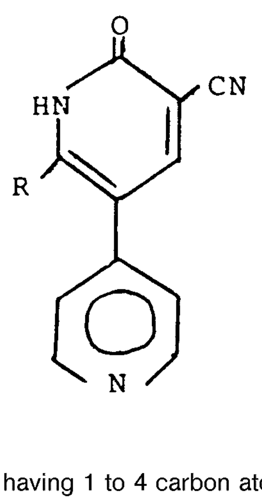

(I)

wherein R is an alkyl group having 1 to 4 carbon atoms, or a solid salt thereof.

## Revendications

1. Composition pharmaceutique pour médicament se présentant sous une forme à dosage par unité et à effet retard en vue d'une administration orale, comprenant plusieurs perles disposées dans une capsule fermée en une matière soluble dans les sucs gastriques, caractérisée en ce que le médicament possède une solubilité dans les sucs intestinaux suffisante pour une libération à effet retard dans ceux-ci et une solubilité notablement plus élevée dans les sucs gastriques et en ce que chacune des perles comporte un noyau inerte, sous forme particulaire, sur lequel adhère un enrobage de particules dudit médicament, cet enrobage de médicament étant entouré par un enrobage, provoquant un effet retard, qui comprend au moins trois polymères mélangés, un premier de ces polymères étant soluble dans les sucs gastriques à toutes les valeurs de pH rencontrées dans le tractus gastrointestinal, un deuxième de ces polymères étant pratiquement insoluble dans les sucs gastriques à des valeurs de pH inférieures à 3, mais soluble dans ceux-ci à des valeurs de pH de 5 et plus et le troisième de ces polymères étant insoluble dans les contenus du tractus gastro-intestinal à toutes les valeurs de pH normalement rencontrées dans ce dernier, les trois polymères étant présents dans des proportions telles qu'ils permettent une libération à effet retard du médicament durant le passage des perles à travers l'estomac et le tractus intestinal.

2. Composition pharmaceutique suivant la revendication 1, caractérisée en ce que le poids du troisième polymère, insoluble, présent dans l'enrobage provoquant un effet retard est supérieur à la somme des poids des deux autres polymères.

3. Composition pharmaceutique suivant la revendication 2, caractérisée en ce que le rapport du poids du troisième polymère à la somme des poids des deux autres polymères est de 3:2 à 2:1.

4. Composition pharmaceutique suivant l'une quelconque des revendications précédentes, caractérisée en ce que le premier polymère constitue 20 % en poids ou moins du mélange de polymères formant l'enrobage provoquant un effet retard.

5. Composition pharmaceutique suivant l'une quelconque des revendications précédentes, caractérisée en ce que le médicament est une pyridyl-(1H)-pyridone répondant à la formule générale :

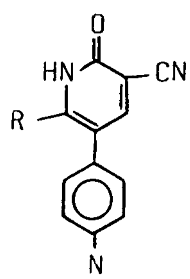

(1)

dans laquelle R est un radical alcoyle comportant 1 à 4 atomes de carbone, ou un sel solide de celle-ci.

6. Composition pharmaceutique suivant l'une quelconque des revendications précédentes, caractérisée en ce que les noyaux inertes des perles se présentent sous la forme de nonpareils.

7. Composition pharmaceutique suivant l'une quelconque des revendications précédentes, dans laquelle les noyaux des perles ont une dimension qui ne dépasse pas la maille de tamis de 707 µm.

8. Composition pharmaceutique suivant la revendication 7, dans laquelle les noyaux des perles ont une dimension comprise entre les mailles de tamis de 595 et 707 µm.

9. Composition pharmaceutique suivant l'une quelconque des revendications précédentes, caractérisée en ce que le premier polymère est choisi parmi l'hydroxypropylméthylcellulose et la polyvinylpyrrolidone.

10. Composition pharmaceutique suivant l'une quelconque des revendications précédentes, caractérisée en ce que le deuxième polymère est le phtalate d'hydroxypropylméthylcellulose.

**11.** Composition pharmaceutique suivant l'une quelconque des revendications précédentes, caractérisée en ce que le troisième polymère est l'éthylcellulose.

**12.** Procédé d'élaboration d'une composition pharmaceutique pour médicament se présentant sous une forme à dosage par unité et à effet retard en vue d'une administration orale, comprenant plusieurs perles disposées dans une capsule fermée en une matière soluble dans les sucs gastriques, caractérisé en ce que le médicament possède une solubilité dans les sucs intestinaux suffisante pour une libération à effet retard dans ceux-ci et une solubilité notablement plus élevée dans les sucs gastriques et en ce que les perles sont préparées :

en enrobant des particules de noyau inertes au moyen d'une suspension de particules dudit médicament et d'un liant servant à faire adhérer les particules de médicament sur les particules de noyau et

à appliquer, sur les particules de noyau enrobées, une solution d'enrobage, provoquant un effet retard, qui comprend au moins trois polymères mélangés, un premier des polymères étant soluble dans les sucs gastriques à toutes les valeurs de pH rencontrées dans le tractus gastrointestinal, un deuxième des polymères étant pratiquement insoluble dans les sucs gastriques à des valeurs de pH inférieures à 3, mais soluble dans ceux-ci à des valeurs de pH de 5 et plus et le troisième des polymères étant insoluble dans les contenus du tractus gastro-intestinal à toutes les valeurs de pH normalement rencontrées dans ce dernier, les trois polymères étant présents dans des proportions telles qu'ils permettent une libération à effet retard du médicament durant le passage des perles à travers l'estomac et le tractus intestinal.

**13.** Procédé suivant la revendication 2, caractérisé en ce que l'enrobage provoquant un effet retard est réalisé en appliquant sur les particules de noyau enrobées de médicament une solution des trois polymères dans un solvant volatil approprié et en faisant s'évaporer le solvant des particules ainsi enrobées.

**14.** Procédé suivant la revendication 13, caractérisé en ce que la solution est réalisée en formant une dispersion des trois polymères dans un milieu convenable, en ajoutant un solvant à bas point d'ébullition à la dispersion et en agitant de façon à obtenir une solution limpide.

**15.** Procédé suivant la revendication 13 ou la revendication 14, caractérisé en ce que l'enrobage provoquant un effet retard est appliqué en plaçant les particules de noyau enrobées de médicament dans une colonne ou une cuvette d'enrobage et en introduisant la solution de polymères dans cette colonne ou cuvette tout en faisant passer un courant d'air à travers les particules de façon à produire des perles enrobées sèches.

**16.** Procédé suivant l'une quelconque des revendications 12 à 15, caractérisé en ce que le médicament est une pyridyl-(1H)-pyridone répondant à la formule générale :

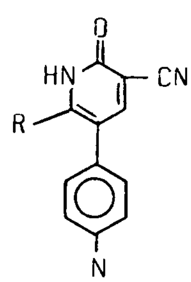

(I)

dans laquelle R est un radical alcoyle comportant 1 à 4 atomes de carbone, ou un sel solide de celle-ci.

**Patentansprüche**

**1.** Arzneimittel in Form einer Einheitsdosis mit verzögerter Freisetzung zur peroralen Verabreichung, enthaltend eine Mehrzahl von Perlen in einer geschlossenen Kapsel aus einem im Magensaft löslichen

Material, **dadurch gekennzeichnet**, daß das Arzneimittel eine genügende Löslichkeit im Darmsaft zur verzögerten Freigabe hierein hat und eine wesentlich höhere Löslichkeit im Magensaft hat, daß jede der Perlen einen inerten Einzelkern hat, dem eine Schicht von Teilchen des Arzneimittels anhaften, diese Arzneimittelschicht von einer Beschichtung mit verzögerter Freigabe umgeben ist, wobei diese Beschichtung ein Gemisch aus mindestens drei Polymeren enthält, das erste Polymere im Magensaft bei allen im Magen-Darm-Trakt auftretenden pH-Werten löslich ist, das zweite Polymere im Magensaft bei einem pH-Wert unterhalb 3 unlöslich ist, jedoch hierin bei einem pH-Wert von 5 und höher löslich ist, und das dritte Polymere im Inhalt des Magen-Darm-Traktes bei allen normalerweise hierin angetroffenen pH-Werten unlöslich ist und daß die drei Polymeren in solchen Anteilen anwesend sind, die es erlauben, eine verzögerte Freigabe des Arzneimittels beim Passieren der Perlen durch den Magen und den Darm-Trakt zu bewirken.

2. Arzneimittel gemäß Anspruch 1, **dadurch gekennzeichnet**, daß das Gewicht des unlöslichen dritten Polymeren in der Beschichtung zur verzögerten Freigabe größer als die Summe der Gewichte der anderen beiden Polymeren ist.

3. Arzneimittel gemäß Anspruch 2, **dadurch gekennzeichnet**, daß das Verhältnis des Gewichts des dritten Polymeren zu der Summe der Gewichtsanteile der anderen beiden Polymeren im Bereich von 3:2 bis 2:1 liegt.

4. Arzneimittel gemäß irgendeinem der vorstehenden Ansprüche, **dadurch gekennzeichnet**, daß das erste Polymere 20 Gew.-% oder weniger des die Beschichtung mit verzögerter Freigabe bildenden Polymerengemischs ausmacht.

5. Arzneimittel gemäß irgendeinem der vorstehenden Ansprüche, **dadurch gekennzeichnet**, daß der Wirkstoff des Arzneimittels ein Pyridyl-(1H)-pyridon der allgemeinen Formel

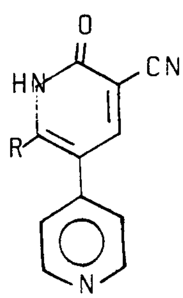

(I)

worin R eine Alkylgruppe mit 1 bis 4 Kohlenstoffatomen ist oder ein festes Salz dieser Verbindung ist.

6. Arzneimittel gemäß irgendeinem der vorstehenden Ansprüche, **dadurch gekennzeichnet**, daß der inerte Kern der Perlen in Form von Nonpareils vorliegt.

7. Arzneimittel gemäß irgendeinem der vorstehenden Ansprüche, **dadurch gekennzeichnet**, daß die Kerne der Perlen eine Teilchengröße von nicht größer als 707 um Maschenweite haben.

8. Arzneimittel gemäß Anspruch 7, **dadurch gekennzeichnet**, daß die Kerne der Perlen eine Teilchengröße von 595 bis 707 um Maschenweite haben.

9. Arzneimittel gemäß irgendeinem der vorstehenden Ansprüche, **dadurch gekennzeichnet**, daß das erste Polymere ausgewählt ist aus der Gruppe Hydroxypropylmethylcellulose und Polyvinylpyrrolidon.

10. Arzneimittel gemäß irgendeinem der vorstehenden Ansprüche, **dadurch gekennzeichnet**, daß das zweite Polymere Hydroxypropylmethylcellulosephthalat ist.

11. Arzneimittel gemäß irgendeinem der vorstehenden Ansprüche, **dadurch gekennzeichnet**, daß das dritte Polymere Ethylcellulose ist.

**12.** Verfahren zur Herstellung eines Arzneimittels in Form einer Einheitsdosis mit verzögerter Freisetzung für perorale Verabreichung, bestehend aus einer Vielzahl von Perlen in einer geschlossenen Kapsel aus einem im Magensaft löslichen Material, **dadurch gekennzeichnet**, daß das Medikament eine genügende Löslichkeit im Darmsaft zur verzögerten Abgabe hierein hat und eine wesentlich größere Löslichkeit im Magensaft hat und daß die Perlen hergestellt sind durch

a) Beschichten von inerten Kernteilchen mit einer Suspension von Teilchen des Arzneimittels und einem Bindemittel zum Binden der Arzneimittelteilchen an den Kernteilchen und

b) Anbringen an diesen beschichteten Kernteilchen einer die verzögerte Freigabe bewirkenden Beschichtungslösung, die ein Gemisch aus mindestens drei Polymeren enthält, wobei das erste Polymere im Magensaft bei allen im Magen-Darm-Trakt auftretenden pH-Werten löslich ist, das zweite Polymere im Magensaft bei einem pH-Wert unter 3 unlöslich, jedoch hierin bei pH-Werten von 5 und größer löslich ist, und das dritte Polymer im Inhalt des Magen-Darm-Traktes bei allen normalerweise hierin angetroffenen pH-Werten unlöslich ist, und die drei Polymeren in solchen Gewichtsanteilen anwesend sind, die es erlauben, eine verzögerte Wirkstoffabgabe während des Passierens der Perlen durch den Magen und den Darmtrakt zu bewirken.

**13.** Verfahren gemäß Anspruch 12, **dadurch gekennzeichnet**, daß die Beschichtung mit verzögerter Freigabe dadurch gebildet wird, daß auf die mit Wirkstoff beschichteten Kernteilchen eine Lösung der drei Polymeren in einem flüchtigen Lösungsmittel hierfür aufgebracht wird und das Lösungsmittel von den so beschichteten Teilchen verdampft wird.

**14.** Verfahren gemäß Anspruch 13, **dadurch gekennzeichnet**, daß die Lösung dadurch gebildet wird, daß eine Dispersion der drei Polymeren in einem geeigneten Medium gebildet wird, ein Lösungsmittel mit niedrigem Siedepunkt zu der Dispersion gegeben wird und zur Bildung einer klaren Lösung das Gemisch gerührt wird.

**15.** Verfahren gemäß Anspruch 13 oder Anspruch 14, **dadurch gekennzeichnet**, daß die Beschichtung mit verzögerter Freigabe dadurch aufgebracht wird, daß man die wirkstoffbeschichteten Kernteilchen in eine Beschichtungskolonne oder -pfanne gibt und die Polymerlösung in die Kolonne oder Pfanne gibt, während ein Luftstrom durch die Teilchen unter Bildung trockener beschichteter Perlen geblasen wird.

**16.** Verfahrengemäß irgendeinem der Ansprüche 12 bis 15, **dadurch gekennzeichnet**, daß der Wirkstoff ein Pyridyl-(1H)-pyridon der allgemeinen Formel

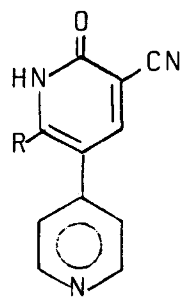

(I)

worin R eine Alkylgruppe mit 1 bis 4 Kohlenstoffatomen ist, oder ein festes Salz hiervon ist.